(19) Europäisches Patentamt / European Patent Office / Office européen des brevets

(11) **EP 2 462 991 A1**

(12) **EUROPEAN PATENT APPLICATION**

(43) Date of publication:
**13.06.2012 Bulletin 2012/24**

(51) Int Cl.:
**A61P 31/04** (2006.01)　　**A61K 31/05** (2006.01)
**A61K 36/63** (2006.01)

(21) Application number: **10194780.2**

(22) Date of filing: **13.12.2010**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **PQS Group B.V.**
**Amsterdam (NL)**

(72) Inventors:
• **Bouter, Pieternella**
  **1019 HC, Amsterdam (NL)**

• **Hendriks, Maikel**
  **1019 HC, Amsterdam (NL)**
• **Kolodziej, Katarzyna**
  **1019 HC, Amsterdam (NL)**

(74) Representative: **Jansen, Bart Antonius Johannes**
**Zacco Netherlands B.V.**
**Nachtwachtlaan 20**
**1058 EA Amsterdam (NL)**

(54) **Composition and use of a fermented olive leaf product for the treatment of nail infections**

(57)　The invention relates to a composition for the treatment of nail infections. The invention also relates to the use of olive leaf product, and oleuropein and derivatives thereof, for the preparation of a product for the treatment of infections, in particular nail infections, more in particular bacterial and/or fungal nail infections. The compositions according to the invention are based on natural products and have a lower chance to induce allergies in users than conventional products.

Figure 1.

**Description**

**FIELD OF THE INVENTION**

The invention relates to a composition for the treatment of nail infections. The invention also relates to the use of fermented olive leaf product for the preparation of a product for the treatment of nail and skin infections.

**BACKGROUND OF THE INVENTION**

Bacterial and fungal nail infections, such as onychomycosis and bacterial and fungal skin infections (such as athlete foot and fungal infections associated with diabetes), are diseases for which various compositions for treatments are known. The formulation of the treatments typically includes one or more active ingredients dissolved in a suitable carrier for nail application. However, the active ingredients used in a number of popular products, either natural or synthetic, frequently comprise allergens and associated health risks.

**OBJECT AND SUMMARY OF THE INVENTION**

The object of the invention is to provide a treatment of nail and skin infections with minimal side effects and/or health risks. It is another object of the invention to provide a composition that is efficient in treating nail infections, improving therapies against nail and/or skin infections and/or prevent nail and/or skin infections.

The invention provides a composition for the treatment of nail and/or infections, in particular bacterial and/or fungal nail infections, comprising at least one olive leaf product. When used in an effective amount, olive leaf products have shown to be efficient in treating and preventing microbial nail infections, as well as positively contributing to therapies against such infections. Olive leaf products are also found to contain minimal side-effects and/or health risks. The olive leave may be used as such, but is preferably processed into a composition suitable for treating nails, such as fluid composition, a cream or a powder. Fluid compositions are preferred. The compositions according to the invention are particularly effective when used on human feet.

Preferably, the composition comprises at least 0.1 % by dry weight of olive leaf material, more preferably at least 1%. Such amounts were found to be particularly effective for the treatment and prevention of nial and/or skin infections. Dry weight of olive leaf material is determined by evaporation all solvents from the composition and substracting of optional other solid materials, and substracting the dried weight from the original weight of the composition before drying.

Preferably, the olive leaf product, as used in the composition according to the invention, is extracted via the fermentation of the leaves of the olive plant, in particular of the leaves of the *Olea europaea* plant species. The active ingredient(s) of the extracted product are present in all parts of the olive plant, in variable nature and concentration: the leaf and fruit being the most high-containing. The fermentation process isolates at least one or more of the active compounds comprised in the olive leaf product. The fermented olive leaf product comprising of at least one or more active compounds demonstrated antimicrobial properties. The fermented olive leaf product is also found to be essentially free of side-effects, and appears to have minimal health risks.

Optionally, the composition may contain preservatives. A preservative that was found to be particularly suitable for the formulations according to the invention is 2-phenoxyethanol, preferably used in a concentration range of about 0.5%-1%. This compound did not only preserve the formulation, but also showed to contribute to the antimicrobial effect of the composition.

The fermented olive leaf product is obtainable from leaf tissue of the olive plant by a fermentation process by heterofermentative lactic acid bacteria (LAB). The industrial importance of LAB is evidenced amongst other features by their generally recognized as safe (GRAS) status. Preferably, the heterofermentative LAB are selected from a group consisting of *Lactobacillus lactis,* in the order of Lactobacillales.

The fermentation process of the olive leaf results in the generation of compounds that show an antimicrobial effect. Of particular interest is oleuropein (CAS 32619-42-4) and derivatives thereof. Derivatives include hydrolised and/or oxydised related compounds, in particular 10-hydroxyoleuropein, ligstroside, and 10-hydroxyligstroside. Oleuropein is a prominent compound in olive cultivars and can reach concentrations of up to 60-90 mg/g of dry matter in leaves, and may be extracted from the leaves. Other compounds isolated by the fermentation process comprise falvinoids, anthocyannins and tannins.

In a preferred embodiment, the fermented olive leaf product also comprises at least one nail-strengthening agent. The addition of the nail strengthening agent contributes to the regeneration and strength of the infected nail or preventively treated healthy nail.

The nail-strengthening agent comprises at least one nail-strenthening agent selected from a group of keratinous compounds, in particular silk compounds, and/or derivatives thereof. A particularly effective keratineous compound is hydrolized slik fibroin, which adds a nail strengthening effect to the formulation and makes the formulation overall more effective against nail infections. Silk is also moisturizing- due to water retention property which restores the skin moisture balance and elasticity.

In a preferred embodiment for the treatment of nails, the olive leaf product comprises at least one nail permeating agent. The addition of a nail-permeating agent improves the anti-infective effect of the formulation.

The nail permeating agents comprises at least one nail permeating agent selected from the group consisting of C1-C4 lactic acid esters and dimethylisorbide. These nail-permeating agents showed significantly enhanced nail permeation for fermented olive leaf products.

It is preferred if the nail-permeating agent comprises dimethylisosorbide. Dimethylisosorbide showed to be particularly effective in enhancing the nail permeation of fermented olive leaf products. It is advantageous if the composition comprises at least 0.5% w/w dimethylisorbide, preferably between 0.5-5% w/w.

In a preferred embodiment, the composition comprises an emulsifying agent. The emulsifying agent assists in evenly spreading the fermented olive leaf product over the surface to be treated. It also assists in stabilizing the formulation and preventing phase separations which may occur during extended storage of fermented olive leaf products.

Preferably the emulsifying agent is selected from the group consisting of pentylene glycol. Emulsifiers in this group proved to be compatible with fermented olive leaf products. Most preferably, the composition comprises pentylene glycol as an emulsifying agent. Pentylene glycol is particularly compatible with fermented olive leaf products, yielding stable homogeneous formulations.

In a preferred embodiment, the composition has a pH below 8. Compositions with a relatively low pH prove to be more effective with respect to the treatment of nail infections than compositions with a higher pH.

Preferably, the composition comprises an effective amount of fermented olive leaf product, a nail-strengthening agent, a nail-permeating agent, and has a pH below 8. This combination has showed to be the most effective against fungal and/or bacterial nail infections.

It is advantageous if the composition is a liquid- or gel-based formulation. Such compositions are most easily applied, for instance using a nail pen applicator. In a preferred embodiment, the nail pen has replaceable tips in order to prevent renewed infection after repeated use or cross-infection through use by different persons.

The invention further provides an olive leaf product for the preparation of a composition for the treatment of nail infections, specifically bacterial and/or fungal nail infections. This use offers the advantages as described above, in particular if the composition is used for the treatment of nail and/or skin infections, in particular, for the treatment of onychomycosis, athlete foot and nail/skin infections associated with diabetes. The composition is a medicament, a nail infection treatment-assisting product, and/or a nail infection preventative product.

The invention also provides the use of oleuropein and derivatives thereof for the treatment of nail infections, in particular bacterial and/or fungal nail infections. Oleuropein proved to be particularly effective against bacterial and/or fungal nail infections, in particular onychomycosis. Usefull derivatives of oleuropein include hydrolysates, in particular acid hydrolysates, more specifically 10-hydroxyoleuropein, ligstroside, and 10-hydroxyligstroside. Oleuropein and derivatives thereof are particularly effective when used on human feet.

## DESCRIPTION OF PREFERRED EMBODIMENTS

### Olive extract

Olive leaf extracts are commercially available, typically for use in cosmetics. The olive leaf extract used in these examples is a 1:5 extract, wherein for every 1 part plant material used, 5 parts solvent is used. Other ratios may also give extracts that are usefull according to the invention. The dry matter content of the extract is about 10-20%. The contents are shown in Table Ia. The olive leaf extract from fermentation of L. lactis, is non-toxic and safe to use.

Table Ia: Commercially available olive leaf extract

| Ingredient | % |
| --- | --- |
| Lactobacillus/Olea europaea (Olive) Leaf Ferment Extract | 98.00-100.00 |
| Methyl Paraben | 0.15-0.22 |
| Dowicill 200 | 0.18-0.22 |
| EDTA*4Na | 0.08-0.12 |

The olive leaf extract is produced via the fermentation of the leaves of the olive plant with the bacteria *lactobacillus lactis.* This fermentation process allows for the isolation of the phytochemicals and phenolic compounds - specifically oleuropein, flavonoids, anthocyanins and tannins which demonstrate antioxidant properties. The extract has a pH in the range of 4.5-6.0. The extract contains oleuropein and hydrolysed derivatives thereof. Typical extraction solvents for olive leaf and fermented olive leaves include water and water/alcohol mixtures, preferably water/ethanol.

Also, a paraben-free olive leaf extract may be prepared, as shown in Table Ib.

Table Ib: Paraben free olive leaf extract.

| Ingredient | % |
| --- | --- |
| Lactobacillus/Olea europaea (Olive) Leaf Ferment Extract | 98.00-100.00 |
| EDTA | 0.08-0.12 |

**Example 1: Nail Pen Formulation**

This example shows a liquid, aqueous composition, comprising the following ingredients by weight:

Formulation A

| Ingredient | % |
| --- | --- |
| Olive leaf extract | 40 |
| dimethylisosorbide | 1,5 |
| pentylene glycol | 10 |
| Water | up to 100% |

The formulation contains at least approximately 0.5% oleuropein (including derived antimicrobial compounds). The dry olive leaf matter content is about 4% in this formulation.

The nail pen formulation showed to be effective in the treatment of onychomycosis when applied in a test system as well as on infected toenails in test persons, showing at least a slowing of growth rates. The used composition could be the extract comprising preservatives, in particular methyl paraben and dawcil200. The preservative-free formulation was somewhat less effective.

Optionally, the nail to be treated before applying the formulation may be filed, preferably using a sterilised nail file in order to prevent re-infection. Good results are obtained if the composition is applied to the nail surface at least twice a day, preferably in the mornings and the evenings, over a period of at least 4 weeks. The solution should cover the entire surface of the affected nail including the underside of the front-most edge. The solution should be allowed to penetrate the nail for at least 1-2 minutes before slipping over any foot wear. After 4 weeks of treatment the application may be reduced to once a day for preventative purposes. Please keep in mind that the affected nail has to grow out, which can take up at least 6 to 9 months. In order to prevent re-infection or nourish the nails, the formulation should be applied at least once a day to non-affected nails.

A preferred way to apply the formulation is by using a hand-held applicator pen. Preferably, the pen has a replacable pen tip. In order to prevent cross-infection or re-infection, the pen is supplied with additional disposable tips to be changed frequently.

Qualitative tests also indicated a positive contribution of the composition in enhancing oral therapy for the treatment of onychomycosis. Also, the reoccurrence of nail infections in successfully treated persons appeared to be lower when these persons continued to regularly treat their toenails with the composition.

**Example 2: nail strengthening formulation**

Formulation B

| Ingredient | % |
| --- | --- |
| lactobacillus olive leaf extract | 40 |

(continued)

| Ingredient | % |
|---|---|
| (parabene free) | |
| dimethylisosorbide | 1.5 |
| phenoxyethanol | 0.7 |
| 2% hydrolised silk fibroin | 2 |
| Water | up to 100% |

The nail pen formulation showed to be effective in the treatment of onychomycosis when applied in a test system as well as on infected toenails in test persons, showing at least a slowing of growth rates. 0.7% phenoxyethanol was added as a preservative, but also showed to contribute to the effectiveness of the formulation as a whole. Qualitative tests also indicated a positive contribution of the composition in enhancing oral therapy for the treatment of onychomycosis. Also, the reoccurrence of nail infections in successfully treated persons appeared to be lower when these persons continued to regularly treat their toenails with the composition, which is an indication of a preventive effect. The addition of hydrolsed silk fibroin adds a nail-strengthening effect. The formulation can be used in a similar way as example 1.

**Example 3: Microbiological tests**

**3.1 Methods**

The different strains that were tested are:

- Candida albicans ATCC 10231
- Pseudomonas aeruginosa ATCC 27853
- Enterococcus hirae ATCC 10541

Of each strain a culture was prepared and the number of cells/ml was determined. The concentration was adjusted to 102 to 103 cfu/ml. The strain was suspended in buffered pepton water. Of each strain 9 ml (102 or 103 cfu/ml) was added to 6 sterile tubes. To each tube 1 ml of the formula according to example 1 or 2 was added. The formula was filter sterilized with filters with a pore size of 0.45 $\mu$m before being added to the strain. The samples were stored for 96 hours, Pseudomonas, Staphylococcus and Enterococcus hirae at 37 °C, Candida and Trichophyton at 25 °C. As a positive control the strains without formula were used. As a negative control the formulas without micro-organisms were used. After 96 hours a sample was taken to determine the amount of cells. This was analyzed by aerobic plate count conform ISO 4833.

**3.2. Results**

The positive controls were all found to have a count of log 6 or 7 after 96 hours. The negative controls were clean. The logarithm was calculated for the amounts after 0 hours and after 96 hours. Values after 0 hours minus values after 96 hours were calculated According to formula I:

$$\text{Log } (a+b) - \text{Log } (c) \qquad\qquad (I)$$

a= number of colony forming units per ml sample after 0 hours
b= number of colony forming units per ml formula after 96 hours (blank)
c = number of colony forming units per ml sample after 96 hours

The tables show per formula the difference in number of cells per ml sample between 0 and 96 hours. The higher the positive values, the more cells were killed. The higher the negative values, the higher the increase in growth in 96 hours and the less effective the product.

Table I: Results for P. aeruginosa

| Result after 0h (a) | Results after 96h 40% olive leaf +parabens (c) | Result after 96h blancs (b) | Result after 96h control (no active ingredient) (c) |
|---|---|---|---|
| 300 | <1 | <1 | 49000000 |
| Result after 0h | Results after 96h 40% olive leaf without preservatives | Result after 96h blancs | Result after 96h control (no active ingredient) |
| 4180 | <10 | <10 | >30000000 |

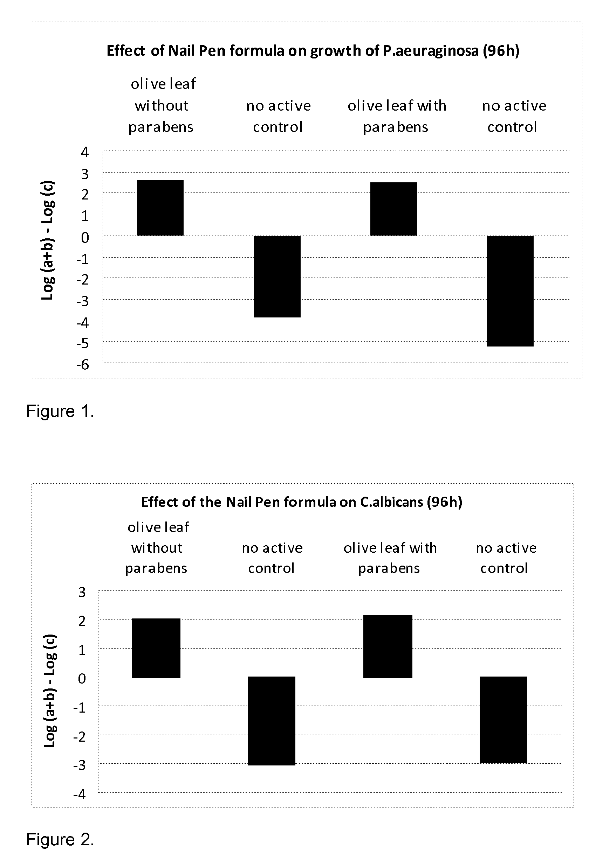

Figure 1 shows the results processed according to formula I. The effect of the nail pen formula was assessed on Pseudomonas aeruginosa in two independent experiment, where 40% active ingredient (olive leaf extract with or without added preservative, as indicated respectively). In both experiments controls: blank (no microorganisms) and negative (no active ingredient formulation) were used. The higher the positive values, the more cells were killed, which is indicative of a beneficial effect. The higher the negative values, the higher the increase in growth in 96 hours and the less effective the product is expected to be.

Table II: Results for C. albicans

| Result after 0h | Results after 96h 40% olive leaf +parabens | Result after 96h blancs | Result after 96h control (no active ingredient) |
|---|---|---|---|
| 400 | 3 | <1 | 373000 |
| Result after 0h | Results after 96h 40% olive leaf without preservatives | Result after 96h blancs | Result after 96h control (no active ingredient) |
| 955 | <10 | <10 | 1150000 |

Figure 2 shows the results processed according to formula I. The effect of the nail pen formula was assessed on Candida albicans in two independent experiments, where 40% active ingredient (olive leaf extract with or without added parabene preservative, as indicated respectively). In both experiments controls were used: blank (no microorganisms) and negative (no active ingredient formulation). The higher the positive values, the more cells were killed, which is indicative of a beneficial effect. The higher the negative values, the higher the increase in growth in 96 hours and the less effective the product is expected to be.

Table III: Results for E.hirae

| Result after 0h | Results after 96h 40% olive leaf +parabens | Result after 96h blancs | Result after 96h control (no active ingredient) |
|---|---|---|---|
| 364 | <1 | <1 | 3820000 |

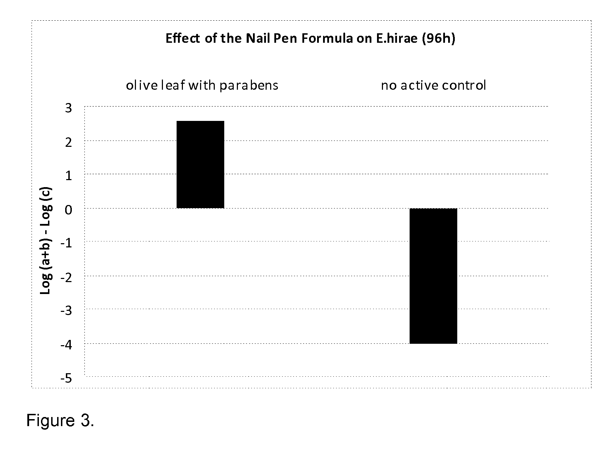

Figure 3 shows the results processed according to formula I. The effect of the nail pen formula was assessed on Enterococcus hirae in two independent experiment, where 40% active ingredient (olive leaf extract with or without added preservative, as indicated respectively). In both experiments controls: blank (no microorganisms) and negative (no active ingredient formulation) were used. The higher the positive values, the more cells were killed, which is indicative of a beneficial effect. The higher the negative values, the higher the increase in growth in 96 hours and the less effective the product is expected to be.

### 3.3. Conclusions

40% of olive leaf extract with or without preservatives was efficiently inhibiting the growth of C. albicans, P.aeruginosa, E.hirae within 96h. The examples illustrate the various ways the teachings of the invention may be embodied for use in medical and non-medical applications, in particular for the treatment and prevention of nail and/or skin infections.

**Claims**

1. Composition for the treatment of nail and/or skin infections comprising an olive leaf product.

2. Composition according to claim 1, wherein the olive leaf product comprises fermented olive leaf product.

3. Composition according to claim 2, wherein the fermented olive leaf product is obtained by a microbial fermentation process, in particular by lactic acid bacteria, preferably Lactobacillus lactis.

4. Composition according to preceeding claims, wherein the olive leaf product comprises oleuropein and/or derivatives thereof.

5. Composition according to any of the preceeding claims wherein the product comprises at least one nail-strengthening agent.

6. Composition according to any of the preceeding claims, wherein the nail-strengthening agent comprises a keratineous compound.

7. Compound according to claim 6, wherein the keratineous compound is hydrolized silk fibroin.

8. Composition according to any of the preceeding claims, wherein the product comprises at least one nail-permeating agent.

9. Composition, according to any of the preceeding claims, wherein the nail-permeating agent comprises of at least one compound, selected from a group, consisting of C1-C4 lactic acid esters and dimethylisosorbide.

10. Composition according to any of the preceeding Claims, wherein the composition has a pH of below 8.

11. Composition according to any of the preceeding claims, wherein the composition comprises a fermented olive leaf product, a nail-strengthening agent, a nail-permeating agent, and has a pH of below 8

12. Composition according to any of the preceding claims, wherein the composition is a liquid or gel-based formulation.

13. Olive leaf composition for the treatment of nail infections, in particular bacterial and/or fungal nail infections.

14. Composition according to Claim 13, wherein the composition is a medicament, a nail infection treatment-assisting product, and/or a nail infection preventive product.

15. Oleuropein and derivatives thereof for the treatment of infections, in particular nail infections, more in particular bacterial and/or fungal nail infections.

**Effect of Nail Pen formula on growth of P.aeuraginosa (96h)**

Figure 1.

**Effect of the Nail Pen formula on C.albicans (96h)**

Figure 2.

Figure 3.

| | Europäisches<br>Patentamt<br>European<br>Patent Office<br>Office européen<br>des brevets | **EUROPEAN SEARCH REPORT** | Application Number<br><br>EP 10 19 4780 |
| --- | --- | --- | --- |

**DOCUMENTS CONSIDERED TO BE RELEVANT**

| Category | Citation of document with indication, where appropriate,<br>of relevant passages | Relevant<br>to claim | CLASSIFICATION OF THE<br>APPLICATION (IPC) |
| --- | --- | --- | --- |
| X | Sravani: "Home Remedies for Athlete's Foot",<br>,<br>11 January 2006 (2006-01-11), XP002627415,<br>Retrieved from the Internet:<br>URL:http://www.drgranny.com/home-remedies/home-remedies-for-athletes-foot/<br>[retrieved on 2011-03-10]<br>* Item 2 (passages bridging pages 1 and 2) * | 1-15 | INV.<br>A61P31/04<br>A61K31/05<br>A61K36/63 |
| X | US 2006/073218 A1 (D AMELIO FRANK S SR [US] ET AL D AMELIO SR FRANK S [US] ET AL)<br>6 April 2006 (2006-04-06)<br>* paragraphs [0020], [0021], [0038], [0071] *<br>* claims 10, 12, 19-24, 30, 34 * | 1-15 | |
| Y | SUDJANA A N ET AL: "Antimicrobial activity of commercial Olea europaea (olive) leaf extract",<br>INTERNATIONAL JOURNAL OF ANTIMICROBIAL AGENTS, ELSEVIER SCIENCE, AMSTERDAM, NL,<br>vol. 33, no. 5, 1 May 2009 (2009-05-01),<br>pages 461-463, XP026073925,<br>ISSN: 0924-8579, DOI:<br>DOI:10.1016/J.IJANTIMICAG.2008.10.026<br>[retrieved on 2009-01-09]<br>*Introduction*<br>* table 1 *<br>-----<br>-/-- | 1-15 | TECHNICAL FIELDS<br>SEARCHED (IPC)<br><br>A61K<br>A61P |

| The present search report has been drawn up for all claims | | |
| --- | --- | --- |
| Place of search<br>Munich | Date of completion of the search<br>10 March 2011 | Examiner<br>Schnack, Anne |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons
 
& : member of the same patent family, corresponding
document

Europäisches
Patentamt

European
Patent Office

Office européen
des brevets

## EUROPEAN SEARCH REPORT

Application Number

EP 10 19 4780

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | MARKIN D ET AL: "In vitro antimicrobial activity of olive leaves = Antimikrobielle Wirksamkeit von Olivenblattern in vitro", MYCOSES, BLACKWELL, BERLIN, DE, vol. 46, 1 January 2003 (2003-01-01), pages 132-136, XP007912524, ISSN: 0933-7407 * page 133, column 2, paragraph 1 - paragraph 3 * *Discussion* | 1-15 | |
| Y | GIAMARELLOS-BOURBOULIS EVANGELOS J ET AL: "Oleuropein: a novel immunomodulator conferring prolonged survival in experimental sepsis by Pseudomonas aeruginosa.", October 2006 (2006-10), SHOCK (AUGUSTA, GA.) OCT 2006 LNKD- PUBMED:16980890, VOL. 26, NR. 4, PAGE(S) 410 - 416, XP002627416, ISSN: 1073-2322 * abstract * | 1-15 | |
| Y | FOSTER K WADE ET AL: "A bipartite interaction between Pseudomonas aeruginosa and fungi in onychomycosis.", November 2005 (2005-11), ARCHIVES OF DERMATOLOGY NOV 2005 LNKD- PUBMED:16301402, VOL. 141, NR. 11, PAGE(S) 1467 - 1468, XP009145759, ISSN: 0003-987X * the whole document * | 1-15 | TECHNICAL FIELDS SEARCHED (IPC) |

The present search report has been drawn up for all claims

| Place of search | Date of completion of the search | Examiner |
|---|---|---|
| Munich | 10 March 2011 | Schnack, Anne |

EPO FORM 1503 03.82 (P04C01)

**EP 2 462 991 A1**

## ANNEX TO THE EUROPEAN SEARCH REPORT
## ON EUROPEAN PATENT APPLICATION NO.

EP 10 19 4780

This annex lists the patent family members relating to the patent documents cited in the above-mentioned European search report.
The members are as contained in the European Patent Office EDP file on
The European Patent Office is in no way liable for these particulars which are merely given for the purpose of information.

10-03-2011

| Patent document cited in search report | Publication date | Patent family member(s) | Publication date |
|---|---|---|---|
| US 2006073218 A1 | 06-04-2006 | NONE | |

EPO FORM P0459